# EUROPÄISCHE PATENTSCHRIFT

(11) **EP 4 302 965 B1**
(45) Veröffentlichungstag und Bekanntmachung des Hinweises auf die Patenterteilung: **26.11.2025**
(21) Anmeldenummer: 23191916.8
(22) Anmeldetag: 17.08.2023
(51) Int. Cl.: B29C 49/42, B29C 49/46, B29C 49/12, B29C 49/36

(54) **VORRICHTUNG UND VERFAHREN ZUM BEHANDELN VON BEHÄLTNISSEN MIT SENSOREINRICHTUNG ZUR FÜLLSTANDSMESSUNG**
DEVICE AND METHOD FOR HANDLING CONTAINERS WITH SENSOR DEVICE FOR LEVEL MEASUREMENT
DISPOSITIF ET PROCÉDÉ DE TRAITEMENT DE RÉCIPIENTS AVEC DISPOSITIF DE DÉTECTION POUR MESURER LE NIVEAU DE REMPLISSAGE

(30) Priorität: 29.08.2022 DE 102022121827
(43) Veröffentlichungstag der Anmeldung: 10.01.2024
(73) Patentinhaber: Krones AG, 93073 Neutraubling (DE)
(72) Erfinder: Soellner, Juergen, 93073 Neutraubling (DE); Theen, Daniel, 93073 Neutraubling (DE); Gerngross, Florian, 93073 Neutraubling (DE); Trauner, Christian, 93073 Neutraubling (DE)
(74) Vertreter: Hannke Bittner & Partner mbB Regensburg

(56) Entgegenhaltungen:
- EP-A1- 2 684 673
- DE-A1- 102013 101 356
- JP-A- 2018 051 870
- JP-A- H0 912 013
- JP-B1- 6 455 574
- US-A1- 2011 232 233
- US-A1- 2012 210 673

## Beschreibung

Die vorliegende Erfindung bezieht sich auf eine Vorrichtung und ein Verfahren zum Behandeln von Behältnissen und insbesondere Kunststoffvorformlingen und/oder Kunststoffbehältnissen. Derartige Vorrichtungen und Verfahren sind aus dem Stand der Technik seit langem bekannt. Bei einer derartigen Behandlung werden die Kunststoffvorformlinge beispielsweise zu Kunststoffbehältnissen umgeformt oder innerhalb einer Fülleinrichtung mit einem fließfähigen Medium, wie beispielsweise einem Getränk, befüllt.

Bei der Umformung werden die Kunststoffvorformlinge dabei üblicherweise innerhalb einer Umformungsstation und genauer innerhalb einer Blasformeinrichtung durch Beaufschlagung mit einem fließfähigen Medium zu den Kunststoffbehältnissen umgeformt. Die Blasformeinrichtungen weisen dabei üblicherweise zwei Blasformseitenteile und ein Bodenteil auf, welche gemeinsam einen Hohlraum ausbilden, welcher einer Negativkontur des fertigen Kunststoffbehältnisses entspricht. Aus dem Stand der Technik sind weiterhin sog. Blasformmaschinen bekannt, welche während des Blasvorgangs die Kunststoffvorformlinge auch in ihrer Längsrichtung dehnen. Hierbei handelt es sich insbesondere um sog. Streckblasmaschinen.

In jüngerer Zeit sind auch Vorrichtungen und Verfahren bekannt geworden, bei denen die Umformung der Kunststoffvorformlinge zu den Kunststoffbehältnissen innerhalb eines Reinraumes erfolgt. Auf diese Weise ist es beispielsweise möglich, dass bereits der gesamte Umformungsprozess unter sterilen Bedingungen erfolgt und daher anschließend die gefertigten Kunststoffbehältnisse nicht erneut oder nur mit verringertem Aufwand sterilisiert werden müssen.

Eine Vielzahl von Umformungsstationen ist dabei an einem bewegbaren und insbesondere drehbaren Transportträger angeordnet, welcher innerhalb des Reinraums angeordnet ist. Der Transportträger ist dabei mittels einer Dichtungseinrichtung, wie insbesondere einem Wasserschloss, gegenüber einem stehenden Teil der Vorrichtung sowie einer unsterilen Umgebung abgedichtet.

Aus dem internen Stand der Technik der Anmeldering ist dabei bekannt, dass die Dichtungseinrichtung einen mit einer Flüssigkeit befüllten Aufnahmeraum aufweist, wobei eine umlaufende drehende Wandung in die Flüssigkeit ragt. Um den Füllstand des Aufnahmeraums zu überwachen, ist dabei direkt an der Dichtungseinrichtung eine Sensoreinrichtung angeordnet. Die Sensoreinrichtung dient dabei insbesondere dazu einen zu niedrigen Füllstand der Flüssigkeit anzuzeigen, da bei einer zu geringen Füllhöhe die Dichtwirkung nicht mehr sichergestellt werden kann.

Enthält der Aufnahmeraum, zu viel Flüssigkeit, beispielswiese durch Kondensatbildung und dergleichen läuft der Aufnahmeraum über. Auch zu hohen Umfangsgeschwindigkeiten des drehbaren Transportträgers können dazu führen, dass Flüssigkeit aus dem Aufnahmeraum überschwappt, da an dem äußeren Ring eine Störkontur entsteht. Beides führt dann dazu, dass die (unter Umständen kontaminierte) Flüssigkeit des Aufnahmeraums unkontrolliert in den Reinraum gelangt.

DE 10 2013 101356 A1, EP 2 684 673 A1, JP 6 455574 B1, US 2012/210673 A1, JP 2018 051870 A, JP H09 12013 A und US 2011/232233 A1 offenbaren Vorrichtungen und Verfahren nach den Oberbegriffen der unabhängigen Ansprüchen.

Der vorliegenden Erfindung liegt daher die Aufgabe zugrunde, eine Vorrichtung und ein Verfahren bereitzustellen, mit welcher / mit welchem die Kontamination des Reinraums mit der Dichtungs-Flüssigkeit verhindert werden kann. Diese Aufgabe wird erfindungsgemäß durch die Gegenstände der unabhängigen Ansprüche erreicht. Vorteilhafte Ausführungsformen und Weiterbildungen sind Gegenstand der Unteransprüche.

Die Erfindung wird durch die Merkmale der unabhängigen Ansprüche definiert.

Durch diese Anordnung wird die oben erwähnte Störkontur an der Außenseite der Dichtungseinrichtung bzw. des Wasserschlosses vermieden, so dass ein Überschwappen durch diese Störkontur ausgeschlossen ist. Der Füllstand der Flüssigkeit innerhalb des Überlaufbehälters ist dabei relativ konstant und wird insbesondere nicht von der Rotation des Transportträgers beeinflusst. Bei einem Anstieg der Flüssigkeitshöhe innerhalb des Überlaufbehälters kann die Flüssigkeit zudem insbesondere gezielt von diesem abgeführt werden und läuft somit nicht mehr unkontrolliert in den Reinraum.

Bei einer bevorzugten Ausführungsform handelt es sich bei der Zulaufleitung um eine Zuführleitung für das flüssige Medium, wobei die Zulaufleitung mit dem Reservoir und dem Überlaufbehälter in Strömungsverbindung steht. Steigt der Füllstand des fließfähigen Mediums innerhalb des Reservoirs an, so kann ein Teil des flüssigen Mediums über die Zulaufleitung in den Überlaufbehälter abfließen und von hier gezielt abgeleitet werden. Dadurch wird ein Überschwappen des flüssigen Mediums aus dem Reservoir verhindert.

Bei einer weiteren bevorzugten Ausführungsform wird daher ein Teil des flüssigen Mediums bei einem Überschreiten eines vorgegebenen Füllstands über eine Überlaufeinrichtung aus dem Überlaufbehälter abgeführt. Besonders bevorzugt wird das flüssige Medium dabei gezielt in eine vorgegebene Richtung bzw. einen vorgegebenen Bereich geführt. Insbesondere wird dadurch verhindert, dass das flüssige Medium unkontrolliert in den Reinraum gelangt. Denkbar wäre auch, dass das flüssige Medium über die Zulaufleitung abgeführt wird.

Bei der Vorrichtung zum Behandeln von Behältnissen handelt es sich bevorzugt um eine Vorrichtung zum Umformen von Kunststoffvorformlingen zu Kunststoffbehältnissen, eine Fülleinrichtung zum Befüllen der Behältnisse, eine Verschließeinrichtung zum Verschließen der Behältnisse, eine Sterilisationseinrichtung zum Sterilisieren der Behältnisse, oder dergleichen.

Bei der Vorrichtung zum Umformen von Kunststoffvorformlingen zu Kunststoffbehältnissen handelt es sich bevorzugt um eine Blasformmaschine. Bei einer vorteilhaften Ausführungsform ist eine Vielzahl von Umformungsstationen an einem gemeinsamen beweglichen Transportträger angeordnet. Bei diesem Träger handelt es sich dabei insbesondere um einen drehbaren Träger und insbesondere ein Blasrad.

Die Umformungsstationen weise dabei jeweils Blasformeinrichtungen auf, welche mehrteilig ausgebildet sind und zwei Blasformseitentele und ein Bodenteil aufweisen. Bevorzugt sind diese Blasformseitenteile lösbar an einer Formträgerschale oder an den Blasformträgern angeordnet. Die Blasformträger sind bezüglich einander schwenkbar, um die Blasformeinrichtungen zu öffnen und zu schließen. Zudem weist der Blasformträger Verriegelungsmechanismen auf, um die Formhälften während des Blasvorgangs gegeneinander zu verriegeln.

Vorteilhaft ist eine Vielzahl derartige Blasformeinrichtungen innerhalb einer Blasmaschine und besonders bevorzugt innerhalb einer Streckblasmaschine angeordnet. Dies bedeutet, dass die Kunststoffvorformlinge durch Beaufschlagung mit Druckluft zu den Kunststoffbehältnissen expandiert werden. Dazu weist die Vorrichtung bevorzugt eine Blasdüse auf, welche an eine Mündung der Kunststoffvorformlinge anlegbar ist, um die Kunststoffvorformlinge so mit Blasluft zu beaufschlagen bzw. mittels Blasluft zu expandieren. Daneben ist bevorzugt auch eine Ventilanordnung vorgesehen, welche die Zuführung der Blasluft an die Kunststoffvorformlinge steuert.

Bei einer weiteren vorteilhaften Ausführungsform weisen die Blasformeinrichtungen bzw. die Umformungsstationen jeweils Reckstangen auf, welche die Kunststoffvorformlinge in ihrer Längsrichtung dehnen. Besonders bevorzugt ist die Blasmaschine bzw. sind der Träger und die Umformungsstationen innerhalb eines Reinraums angeordnet, welcher die Blasformmaschine gegenüber einer unsterilen Umgebung abgrenzt. Antriebseinrichtungen für die Verschließung, Verriegelung und/oder Öffnung der Blasformen befinden sich dabei bevorzugt außerhalb des Reinraums angeordnet.

Die Umformungsstationen werden dabei bevorzugt innerhalb des Reinraums transportiert, wobei der Reinraum bevorzugt von mehreren Wandungen begrenzt wird. Bevorzugt wird der Reinraum dabei von wenigstens einer stehenden Wandung und einer sich gegenüber dieser stehenden Wandung bewegenden Wandung begrenzt. Dabei kann beispielsweise der Transportträger, an dem die Umformungsstationen und/oder Blasformeinrichtungen angeordnet sind, bereits eine dieser Wandungen und insbesondere die sich bewegende Wandung aufweisen bzw. ausbilden. Der Reinraum grenzt die Umformungsstationen und/oder Blasformeinrichtungen dabei insbesondere von einer unsterilen Umgebung ab.

Bei einem weiteren bevorzugten Verfahren werden Teile der Blasformeinrichtung temperiert. Insbesondere werden Teile der Blasformeinrichtung erwärmt. Bevorzugt erfolgt dabei diese Erwärmung mittels elektrischer Energie oder mittels eines fließfähigen und insbesondere flüssigen Temperiermediums. So kann beispielsweise heißes Öl oder Wasser verwendet werden, um die Seitenteile der Blasform und/oder das Bodenteil der Blasform zu temperieren. Dabei kann dieses Temperieren mittels Kanälen erfolgen, welche in der Blasform selbst angeordnet sind und/oder auch mittels Kanälen, welche in Blasformschalen angeordnet sind und/oder auch in einem Blasformträger.

Bei einer bevorzugten Ausführungsform ist das Reservoir in Form eines umlaufenden Kanals ausgebildet. Dieser umlaufende Kanal des Reservoirs ist dabei über die Zulaufleitung mit einem flüssigen Medium, wie insbesondere Wasser befüllbar.

Bei einer weiteren bevorzugten Ausführungsform weist die Dichtungseinrichtung eine umlaufende Wandung auf, welche in den umlaufenden Kanal ragt, wobei diese umlaufende Wandung bevorzugt drehbar ausgebildet ist. Die Dichtungsvorrichtung dichtet dabei bevorzugt den Reinraum gegenüber einer unsterilen Umgebung ab. Insbesondere wird dabei durch die Dichtungseinrichtung verhindert, dass unsterile Umgebungsluft und Keime in den Reinraum gelangen und diesen kontaminieren.

Bei der umlaufenden Wandung, welche in den umlaufenden Kanal ragt, handelt es sich bevorzugt um wenigstens ein Schwert, welches einen Bestandteil des Wasserschlosses, bzw. der hydraulischen Dichtung bildet. Besonders bevorzugt taucht diese Wandung in die in dem Kanal befindliche Flüssigkeit. Dabei gehört dieses Schwert üblicherweise zu einem drehenden Teil der Anlage und der umlaufende Kanal ist stationär angeordnet.

Die umlaufende Wandung der Dichtungseinrichtung ist demnach bevorzugt drehbar bezüglich des Kanals ausgebildet. Bevorzugt ist dabei die Drehbewegung dieser Wandung an die Drehbewegung des Transportträgers gekoppelt.

Bevorzugt ist der umlaufende Kanal durch die umlaufende Wandung der Dichtungseinrichtung in einem radial inneren Kanalabschnitt und einem radial äußeren Kanalabschnitt unterteilt, wobei das Schwert bevorzugt in einen Bereich zwischen dem inneren Kanalabschnitt und dem äußeren Kanalabschnitt in die Flüssigkeit eintaucht.

Die Sensoreinrichtung steht bevorzugt mit dem flüssigen Medium innerhalb des Überlaufbehälters in Kontakt. Bei einer bevorzugten Ausführungsform gibt die Sensoreinrichtung ein Signal aus, wenn die Sensoreinrichtung nicht mehr in Kontakt mit dem flüssigen Medium ist. Die Sensoreinrichtung zeigt demnach bevorzugt an, wenn der Füllstand des flüssigen Mediums zu niedrig ist, da bei einem zu niedrigen Füllpegel keine Abdichtung mehr sichergestellt werden kann.

Bei einer bevorzugten Ausführungsform wird der umlaufende Kanal dabei bevorzugt über die Zulaufleitung automatisch wieder mit flüssigem Medium gefüllt, sobald die Sensoreinrichtung erkennt, dass der Füllstand zu niedrig ist.

Bei einer weiteren bevorzugten Ausführungsform wird das flüssige Medium bei einem Überschreiten eines vorgegebenen Füllstands über eine Überlaufeinrichtung aus dem Überlaufbehälter abgeführt. Wie oben bereits erwähnt, wird das flüssige Medium dabei auf einen Bereich der Vorrichtung gerichtet, welcher sich außerhalb des Reinraums befindet, so dass dieser durch das flüssige Medium nicht mit Keimen kontaminiert wird. Besonders bevorzugt sind die Sensoreinrichtung und der Überlaufbehälter daher außerhalb des Reinraums angeordnet.

Dabei ist die oben beschriebene Vorrichtung insbesondere dazu eingerichtet und dafür vorgesehen, das beschriebene Verfahren durchzuführen, d. h., dass alle für die obig beschriebene Vorrichtung ausgeführten Merkmale ebenso für das hier beschriebene Verfahren offenbart sind und umgekehrt.

Weitere Vorteile und Ausführungsformen ergeben sich aus der beigefügten Zeichnung.

Darin zeigt:
- Fig. 1: eine schematische Darstellung einer Vorrichtung zum Behandeln von Behältnissen;
- Fig. 2: eine schematische Darstellung der erfindungsgemäßen Sensoreinrichtung; und
- Fig. 3: eine schematische Darstellung der Dichtungseinrichtung.

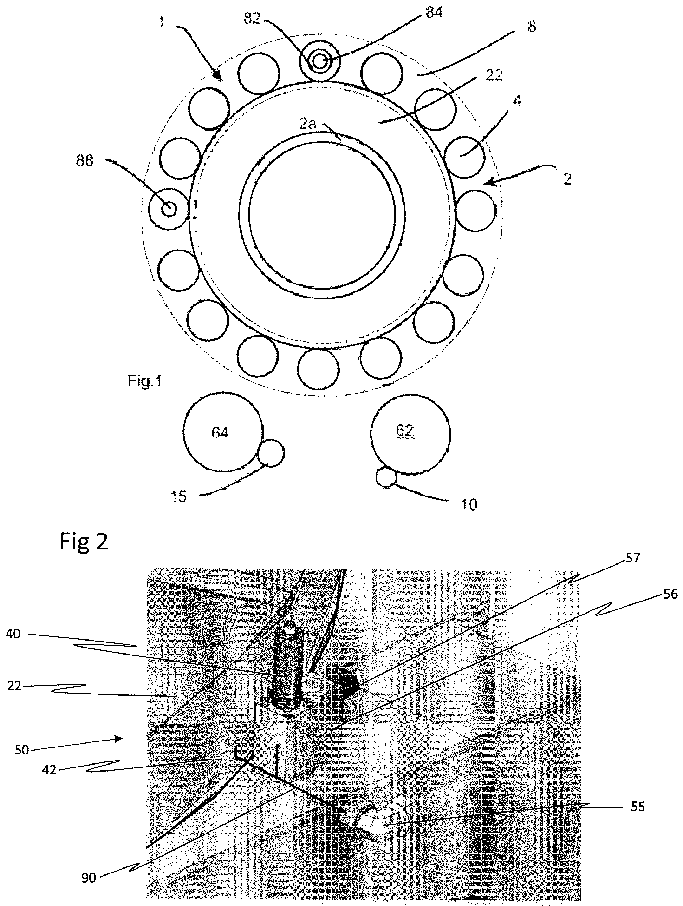

Figur 1 zeigt eine Vorrichtung 1 zum Behandeln von Behältnissen und insbesondere Kunststoffbehältnissen und/oder Kunststoffvorformlingen. In dieser Vorrichtung 1 werden die Kunststoffvorformlinge 10 über eine Zuführeinrichtung 62 der Vorrichtung 1 zugeführt und nach der Umformung werden die Kunststoffbehältnisse 15 über eine Abführeinrichtung 64 von der Vorrichtung 1 abgeführt. Bei dieser Vorrichtung handelt es sich hier demnach um eine Vorrichtung 1 zum Umformen von Kunststoffvorformlingen 10 zu Kunststoffbehältnissen 15.

Die Vorrichtung 1 weist dabei einen drehbaren Träger 22 auf, an dem eine Vielzahl von Behandlungsstationen 4, insbesondere Umformungsstationen angeordnet ist. Diese einzelnen Behandlungs- bzw. Umformungsstationen weisen jeweils Blasformeinrichtungen 82 auf, die in ihrem Inneren einen Hohlraum zum Expandieren der Kunststoffvorformlinge ausbilden.

Das Bezugszeichen 84 kennzeichnet eine Beaufschlagungseinrichtung, welche zum Expandieren der Kunststoffvorformlinge 10 dient. Das Bezugszeichen 88 kennzeichnet eine Reckstange, die dazu dient, die Kunststoffvorformlinge in ihrer Längsrichtung zu dehnen. Bevorzugt weisen sämtliche Umformungsstationen derartige Blasformen 82 sowie Reckstangen 88 auf. Das fließfähige Medium wird dabei über mindestens einen Ringkanal 2a über (nicht dargestellte) Leitungen zu den einzelnen Behandlungsstationen geführt.

Das Bezugszeichen 8 kennzeichnet schematisch einen Reinraum, der hier bevorzugt ringförmig ausgebildet ist und den Transportpfad der Kunststoffvorformlinge 10 umgibt. Bevorzugt ist eine (geometrische) Drehachse bezüglich derer der Transportträger drehbar ist, außerhalb des Reinraums 8 angeordnet. Bevorzugt ist der Reinraum mit einer Dichtungseinrichtung, welche bevorzugt wenigstens zwei Wasserschlösser aufweist, gegenüber der unsterilen Umgebung abgedichtet.

Figur 2 zeigt eine schematische Darstellung der erfindungsgemäßen Sensoreinrichtung 40. Die Sensoreinrichtung 40 ist dabei an einem Überlaufbehälter 56 mit Überlaufeinrichtung 57, über welche das flüssige Medium abgeführt werden kann, angeordnet.

Das Reservoir 42 für das flüssige Medium sowie der Überlaufbehälter 56 und/oder die Sensoreinrichtung 40 sind dabei mit einer Zulaufleitung 55 für das flüssige Medium in Strömungsverbindung 90. Das Bezugszeichen 50 kennzeichnet die Dichtungseinrichtung, welche den drehbaren Transportträger 22 von einem stehenden Teil der Vorrichtung abdichtet bzw. den Reinraum in welchen der Transportträger 22 angeordnet ist, von der Umgebung abgrenzt.

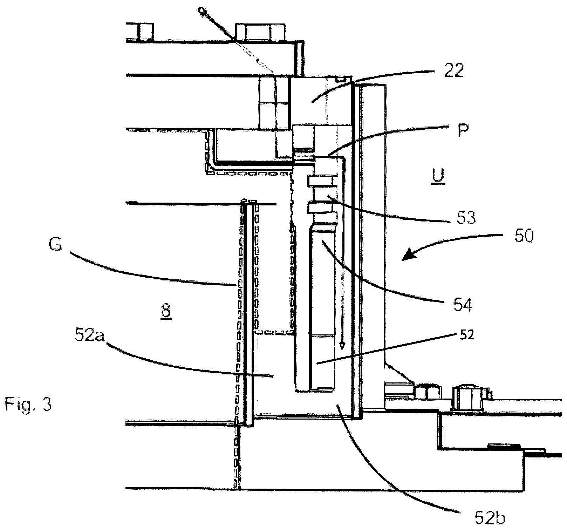

Fig. 3 zeigt eine Detaildarstellung der Dichtungseinrichtung 50. Dabei ist ein umlaufender Kanal 52 dargestellt, in dem sich eine Flüssigkeit wie etwa Wasser (nicht gezeigt) befindet.

Dieser umlaufende Kanal 52 ist hier stationär bzw. gehört zu dem stationären Teil der Vorrichtung.

In den Kanal 52 taucht eine umlaufende Wandung 54 bzw. ein Schwert ein. Diese umlaufende Wandung 54 ist bevorzugt drehbar ausgebildet bzw. bildet einen Bestandteil des drehenden Teils der Vorrichtung. Bevorzugt ist die umlaufende Wandung 54 an dem Transportträger 22 angeordnet.

Durch diese umlaufende Wandung 54 wird der Kanal 52 in einen ersten bzw. inneren Teilkanal 52a und einen äußeren bzw. zweiten Teilkanal 52b unterteilt. Dabei bildet der innere Teilkanal 52a bevorzugt noch einen Bereich des Reinraums 8, während der äußere Teilkanal 52b einen Bereich der (unsterilen) Umgebung U bildet. Die gestrichelte Linie G kennzeichnet die Grenze des Reinraums 8.

### Bezugszeichenliste

- 1: Vorrichtung
- 2: Transporteinrichtung
- 2a: Ringkanal
- 4: Behandlungsstationen
- 8: Reinraum
- 10: Kunststoffvorformling
- 15: Behältnis
- 22: Transportträger
- 40: Sensoreinrichtung
- 42: Reservoir
- 50: Dichtungseinrichtung
- 52: umlaufender Kanal
- 52a: innere Teilkanal
- 52b: äußere Teilkanal
- 54: umlaufende Wandung
- 55: Zulaufleitung
- 56: Überlaufbehälter
- 57: Überlaufeinrichtung
- 62: Zuführeinrichtung
- 64: Abführeinrichtung
- 82: Blasformeinrichtung
- 84: Beaufschlagungseinrichtung
- 88: Reckstange
- 90: Strömungsverbindung

- U: Umgebung
- G: Grenze des Reinraums 8

## Patentansprüche

1. Vorrichtung (1) zum Behandeln von Behältnissen (15) mit einer Transporteinrichtung (2), welche die zu behandelnden Behältnisse (15) entlang eines vorgegebenen Transportpfads transportiert, wobei diese Transporteinrichtung (2) einen drehbaren Transportträger (22) aufweist, an dem eine Vielzahl von Behandlungsstationen (4) zum Behandeln der Behältnisse (15) angeordnet ist, wobei die Vorrichtung (1) einen Reinraum (8) aufweist, innerhalb dessen die Behältnisse (15) in vorgegebener Weise behandelt werden, und mit einer Dichtungseinrichtung (50), welche diesen Reinraum (8) gegenüber einer Umgebung abdichtet, wobei diese Dichtungseinrichtung (50) ein mit einem flüssigen Medium befüllbares Reservoir (42) aufweist und mit einer Sensoreinrichtung (40) zum wenigstens mittelbaren Messen eines Füllstandes dieses flüssigen Mediums,
**dadurch gekennzeichnet, dass**
die Sensoreinrichtung (40) in einer mit dem Reservoir (42) in Strömungsverbindung bringbaren Zulaufleitung (55) angeordnet ist, wobei es sich bei der Zulaufleitung (55) um eine Zuführleitung für das flüssige Medium handelt, wobei die Zulaufleitung (55) mit dem Reservoir (42) und einem Überlaufbehälter (56) in Strömungsverbindung steht.

2. Vorrichtung (1) nach Anspruch 1,
**dadurch gekennzeichnet, dass**
die Sensoreinrichtung (40) an dem Überlaufbehälter (56) angeordnet ist.

3. Vorrichtung (1) nach Anspruch 2,
**dadurch gekennzeichnet, dass**
ein Teil des flüssigen Mediums bei einem Überschreiten eines vorgegebenen Füllstands über eine Überlaufeinrichtung (57) aus dem Überlaufbehälter (56) abgeführt wird.

4. Vorrichtung (1) nach Anspruch 1,
**dadurch gekennzeichnet, dass**
das Reservoir (42) in Form eines umlaufenden Kanals (52) ausgebildet ist.

5. Vorrichtung (1) nach Anspruch 4,
**dadurch gekennzeichnet, dass**
die Dichtungseinrichtung (50) eine umlaufende Wandung (54) aufweist, welche in den umlaufenden Kanal (52) ragt, wobei diese umlaufende Wandung (54) bevorzugt drehbar ausgebildet ist.

6. Vorrichtung (1) nach wenigstens einem der vorhergehenden Ansprüche,
**dadurch gekennzeichnet, dass**
die Sensoreinrichtung (40) ein Signal ausgibt, wenn die Sensoreinrichtung (40) nicht mehr in Kontakt mit dem flüssigen Medium ist.

7. Vorrichtung (1) nach wenigstens einem der vorhergehenden Ansprüche,
**dadurch gekennzeichnet, dass**
der umlaufende Kanal automatisch wieder mit flüssigem Medium gefüllt wird, sobald die Sensoreinrichtung erkennt, dass der Füllstand zu niedrig ist.

8. Vorrichtung (1) nach wenigstens einem der vorhergehenden Ansprüche,
**dadurch gekennzeichnet, dass**
die Sensoreinrichtung (40) und der Überlaufbehälter (56) außerhalb des Reinraums (8) angeordnet sind.

9. Verfahren zum Behandeln von Behältnissen (15), wobei eine Transporteinrichtung (2) die zu behandelnden Behältnisse (15) entlang eines vorgegebenen Transportpfads transportiert, und diese Transporteinrichtung (2) einen drehbaren Transportträger (22) aufweist, an dem eine Vielzahl von Behandlungsstationen (4) zum Behandeln der Behältnisse (15) angeordnet ist, mit einem Reinraum (8), innerhalb dessen die Behältnisse (15) in vorgegebener Weise behandelt werden, und mit einer Dichtungseinrichtung (50), welche diesen Reinraum (8) gegenüber einer Umgebung abdichtet, wobei diese Dichtungseinrichtung (50) ein mit einem flüssigen Medium befülltes Reservoir (42) aufweist und eine Sensoreinrichtung (40) wenigstens zeitweise einen Füllstand dieses flüssigen Mediums in diesem Reservoir (42) bestimmt,
**dadurch gekennzeichnet, dass**
die Sensoreinrichtung (40) in einer wenigstens zeitweise mit dem Reservoir (42) in Strömungsverbindung stehenden Zulaufleitung (55) angeordnet ist, wobei es sich bei der Zulaufleitung (55) um eine Zuführleitung für das flüssige Medium handelt, wobei die Zulaufleitung (55) mit dem Reservoir (42) und einem Überlaufbehälter (56) in Strömungsverbindung steht.

## Claims

1. An apparatus (1) for treating containers (15) with a transport device (2) which transports the containers (15) to be treated along a predetermined transport path, wherein said transport device (2) has a rotatable transport carrier (22) at which a plurality of treatment stations (4) for treating the containers (15) is arranged, wherein the apparatus (1) has a clean room (8) within which the containers (15) are treated in a predetermined manner, and with a sealing device (50) which seals said clean room (8) from an environment, wherein said sealing device (50) has a reservoir (42) which can be filled with a liquid medium, and with a sensor device (40) for measuring a filling level of said liquid medium at least indirectly,
**characterized in that**
the sensor device (40) is arranged in a supply line (55) which can be brought into flow connection with the reservoir (42), wherein the supply line (55) is a supply line for the liquid medium, wherein the supply line (55) is in flow connection with the reservoir (42) and an overflow container (56).

2. The apparatus (1) according to claim 1,
**characterized in that**
the sensor device (40) is arranged on the overflow container (56).

3. The apparatus (1) according to claim 2,
**characterized in that**
a portion of the liquid medium is discharged from the overflow container (56) via an overflow device (57) if a predetermined filling level is exceeded.

4. The apparatus (1) according to claim 1,
**characterized in that**
the reservoir (42) is designed in the form of a circumferential channel (52).

5. The apparatus (1) according to claim 4,
**characterized in that**
the sealing device (50) has a circumferential wall (54) which projects into the circumferential channel (52), wherein said circumferential wall (54) is preferably designed to be rotatable.

6. The apparatus (1) according to at least one of the preceding claims,
**characterized in that**
the sensor device (40) outputs a signal if the sensor device (40) is no longer in contact with the liquid medium.

7. The apparatus (1) according to at least one of the preceding claims,
**characterized in that**
the circumferential channel is automatically filled with liquid medium again as soon as the sensor device detects that the filling level is too low.

8. The apparatus (1) according to at least one of the preceding claims,
**characterized in that**
the sensor device (40) and the overflow container (56) are arranged outside the clean room (8).

9. A method for treating containers (15), wherein a transport device (2) transports the containers (15) to be treated along a predetermined transport path, and said transport device (2) has a rotatable transport carrier (22) at which a plurality of treatment stations (4) for treating the containers (15) is arranged, with a clean room (8) within which the containers (15) are treated in a predetermined manner, and with a sealing device (50) which seals said clean room (8) from an environment, wherein said sealing device (50) has a reservoir (42) filled with a liquid medium, and a sensor device (40) determines a filling level of said liquid medium in said reservoir (42) at least at times,
**characterized in that**
the sensor device (40) is arranged in a supply line (55) which is at least at times in flow connection with the reservoir (42), wherein the supply line (55) is a supply line for the liquid medium, wherein the supply line (55) is in flow connection with the reservoir (42) and an overflow container (56).

## Revendications

1. Dispositif (1) de traitement de récipients (15) avec un système de transport (2), qui transporte les récipients (15) à traiter le long d'une voie de transport prédéfinie, dans lequel ledit système de transport (2) présente un support de transport rotatif (22) sur lequel est disposée une pluralité de stations de traitement (4) pour traiter les récipients (15), dans lequel le dispositif (1) présente une salle blanche (8) à l'intérieur de laquelle les récipients (15) sont traités de manière prédéfinie, et avec un système d'étanchéité (50) qui assure l'étanchéité de ladite salle blanche (8) par rapport à un environnement, dans lequel ledit système d'étanchéité (50) présente un réservoir (42) remplissable d'un milieu liquide et avec un système capteur (40) pour la mesure au moins indirecte d'un niveau de remplissage dudit milieu liquide,
**caractérisé en ce que**
le système capteur (40) est disposé dans une conduite d'arrivée (55) apte à être mise en communication fluidique avec le réservoir (42), dans lequel la conduite d'arrivée (55) est une conduite d'alimentation pour le milieu liquide, dans lequel la conduite d'arrivée (55) est en communication fluidique avec le réservoir (42) et un contenant de trop-plein (56).

2. Dispositif (1) selon la revendication 1,
**caractérisé en ce que**
le système capteur (40) est disposé sur le contenant de trop-plein (56).

3. Dispositif (1) selon la revendication 2,
**caractérisé en ce que**
une partie du milieu liquide est évacuée du contenant de trop-plein (56) par un système de trop-plein (57) en cas de dépassement d'un niveau de remplissage prédéfini.

4. Dispositif (1) selon la revendication 1,
**caractérisé en ce que**
le réservoir (42) est réalisé sous la forme d'un canal périphérique (52).

5. Dispositif (1) selon la revendication 4,
**caractérisé en ce que**
le système d'étanchéité (50) présente une paroi périphérique (54) qui dépasse dans le canal périphérique (52), dans lequel ladite paroi périphérique (54) est de préférence réalisée de manière rotative.

6. Dispositif (1) selon au moins l'une quelconque des revendications précédentes,
**caractérisé en ce que**
le système capteur (40) émet un signal lorsque le système capteur (40) n'est plus en contact avec le milieu liquide.

7. Dispositif (1) selon au moins l'une quelconque des revendications précédentes,
**caractérisé en ce que**
le canal périphérique est automatiquement de nouveau rempli de milieu liquide dès que le système capteur détecte que le niveau de remplissage est trop bas.

8. Dispositif (1) selon au moins l'une quelconque des revendications précédentes,
**caractérisé en ce que**
le système capteur (40) et le contenant de trop-plein (56) sont disposés à l'extérieur de la salle blanche (8).

9. Procédé de traitement de récipients (15), dans lequel un système de transport (2) transporte les récipients (15) à traiter le long d'une voie de transport prédéfinie, et ledit système de transport (2) présente un support de transport rotatif (22) sur lequel est disposée une pluralité de stations de traitement (4) pour traiter les récipients (15), avec une salle blanche (8) à l'intérieur de laquelle les récipients (15) sont traités de manière prédéfinie, et avec un système d'étanchéité (50), qui assure l'étanchéité de ladite salle blanche (8) par rapport à un environnement, dans lequel ledit système d'étanchéité (50) présente un réservoir (42) rempli d'un milieu liquide et un système capteur (40) détermine au moins temporairement un niveau de remplissage dudit milieu liquide dans ledit réservoir (42),
**caractérisé en ce que**
le système capteur (40) est disposé dans une conduite d'arrivée (55) en communication fluidique au moins temporairement avec le réservoir (42), dans lequel la conduite d'arrivée (55) est une conduite d'alimentation pour le milieu liquide, dans lequel la conduite d'arrivée (55) est en communication fluidique avec le réservoir (42) et un contenant de trop-plein (56).
